# EUROPEAN PATENT APPLICATION

(11) **EP 2 990 034 A1**
(43) Date of publication of application: **02.03.2016**
(21) Application number: 13883109.4
(22) Date of filing: 22.04.2013
(51) Int. Cl.: A61K 31/14, A61K 33/38, A61P 1/04, A61P 11/02, A61P 15/00, A61P 17/02, A61P 27/02, A61P 31/04, A61P 31/02, B82Y 5/00

(54) **ANTISEPTIC VETERINARY FORMULATION AND METHODS FOR USE THEREOF**

(71) Applicant: Obshchestvo S Ogranichennoy Otvetstvennostyu "Nanobiotekh", Moscow 123103 (RU); Denisov, Albert Nikolaevich, Barnaul 656002 (RU)
(72) Inventor: DENISOV, Albert Nikolaevich, Barnaul Altaiskiy kray 565002 (RU); KRUTYAKOV, Yuriy Andreevich, Moscow 125057 (RU); KUDRINSKY, Aleksey Aleksandrovich, Moscow 119361 (RU)
(74) Representative: AMMANN PATENTANWÄLTE AG BERN
(86) International application number: PCT/RU2013/000343
(87) International publication number: WO 2014/175757

(57) **Abstract**

The invention relates to veterinary science, in particular, to antiseptic formulations with a broad spectrum of activity, and concerns a drug for treating wound surfaces, inflamed mucosa, and postoperative sutures. The invention can be used for prophylaxis of postoperative complications, for treatment and prophylaxis of mastitis and endometritis, conjunctivitis, gingivitis, diarrhea, rhinitis, and otitis in pets and farm animals, *inter alia,* large and small cattle, poultry, cats and dogs. The antiseptic veterinary formulation comprises nanosized silver particles and benzyl-dimethyl-[3-(myristoylamino)propyl]-ammonium chloride. The methods for treating and prophylaxis of mastitis and endometritis, conjunctivitis, gingivitis, diarrhea, rhinitis, and otitis in pets and farm animals comprise the use of the antiseptic veterinary formulation comprising nanosized silver particles and benzyl-dimethyl-[3-myristoylamino)propyl]-ammonium chloride.

## Description

### Field of technology

The invention relates to veterinary science, in particular, to antiseptic formulations with a broad spectrum of activity, and concerns a drug for treating wound surfaces, inflamed mucosa, and post-surgical sutures. The invention can be used for prophylaxis of postoperative complications, for treatment and prophylaxis of mastitis and endometritis, conjunctivitis, gingivitis, diarrhea, rhinitis, and otitis in pets and farm animals, *inter alia*, large and small cattle, poultry, cats and dogs.

### Background art

It is known that the treatment of infectious diseases in animals is often a very time-consuming task, and the use of many conventional antibacterial drugs that have toxic effects on the liver, kidneys and intestines is not effective enough. Treatment of such infections is not only great difficulty due to the complexity of its implementation, but the high cost of used medicines, such as antibiotics.

In this regard, the treatment of infectious diseases in animals generates a need for cost-effective and, thus, sufficiently effective antiseptics.

The prior art (Russian Federation patents No. 2161961 from January 20, 2001, No. 2164135 of March 20, 2001, No. 2234313 of August 20, 2004) discloses antibacterial preparations based on cationic surface active agents (SAAs) on the basis of quaternary ammonium compounds. The disadvantage of these preparations is the need to use rather concentrated solutions to achieve a disinfecting effect, for example, 0 to 0.1 wt% benzyl-dimethyl-[3-myristoylamino)propyl]-ammonium chloride (Russian Federation patent Nº 2164135 of March 20, 2001).

The prior art also discloses antibacterials based on colloidal silver. The disadvantage of these preparations is also the need to use rather concentrated silver sols to achieve a disinfecting effect, for example, 0.1 wt% to 0.4 wt% for the drug Argovit (Russian Federation patent Nº 2342120 of December 27, 2008) based on the silver content in the reconstituted formulation.

The prior art also discloses antibacterials based on colloidal silver and cationic SAA. Russian Federation patent Nº 2419439 of May 27, 2011 discloses an antibacterial agent based on colloidal silver suspended in water, characterized in that it also contains at least one cationic surface acting agent. Russian Federation patent Nº 2427380 of August 27, 2011 disclosed a disinfectant for the skin containing colloidal silver, at least one cationic surface acting agent, excipients and water in the following ratio (wt%): colloidal silver 10⁻⁵-0.5; cationic surface acting agent 0.01 -25; water and additive agents up to 100. Russian Federation patents No. 2419439 of May 27, 2011 and No. 2427380 of August 27, 2011 indicated that the preparations containing colloidal silver and cationic surface acting agents have significantly greater antibacterial activity than the corresponding silver sols and SAA. However, the use of such preparations for the treatment of infectious diseases in animal requires the development of optimum route of administration.

In this regard, a need arises to increase the efficiency of treatment of infectious diseases in animals through the use of preparations containing colloidal silver and SAAs.

This technical result is achieved through using an antiseptic veterinary formulation described in more detail below.

### Disclosure of the invention

The essential features of the invention disclosed by Russian Federation patent No. 2419439 of May 27, 2011 is the closest analogue of the claimed invention.

During the experimental studies, it was found that a preparation containing colloidal silver in the form of nanosized particles and benzyl-dimethyl-[3-(myristoylamino)propyl]-ammonium chloride was effective in the prophylaxis of postoperative complications, in the treatment and prophylaxis of mastitis and endometritis, conjunctivitis, gingivitis, rhinitis, otitis in pets and farm animals including cattle, birds, cats, dogs, and also demonstrated a wound healing effect. Using nanosized silver particles or nanosized silver particles in combination with silver salts and benzyldimethyl-[3-(myristoylamino)propyl]-ammonium chloride reduces the treatment time and decreases the incidence of complications. Using nanosized particles of silver or silver nanoparticles in combination with silver salts and benzyl-dimethyl-[3-(myristoylamino)propyl]-ammonium chloride can also expand the range of antiseptic veterinary preparations for the prevention of postoperative complications, treatment and prevention of mastitis and endometritis, conjunctivitis, gingivitis, rhinitis, otitis in pets and farm animals, including cattle, birds, cats, dogs.

The claimed invention relates to the antiseptic veterinary preparations for prophylaxis of postoperative complications, treatment and prophylaxis of mastitis and endometritis, conjunctivitis, gingivitis, rhinitis, otitis in pets and farm animals, including large and small cattle, poultry, cats and dogs, and also for the treatment of mucous membranes, open wounds and damaged skin.

The claimed invention relates to the antiseptic veterinary formulation containing nanosized silver particles and benzyl-dimethyl-[3-(myristoylamino)propyl]-ammonium chloride.

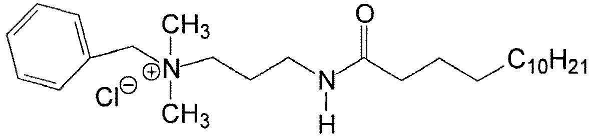

In the preferred embodiment of the invention, the concentration of benzyl-dimethyl-[3-(myristoylamino)propyl]-ammonium chloride in the antiseptic veterinary formulation ranges from 0.002 to 0.05 wt%.

In the preferred embodiment of the invention, the concentration of nanosized silver particles in the antiseptic veterinary formulation ranges from 0.1 to 100 µg/ml.

In the preferred embodiment of the invention, the antiseptic veterinary formulation contains at least one silver salt.

In the preferred embodiment of the invention, the silver salt is selected from the group which comprises nitrate, acetate, citrate, chloride, bromide, and iodide silver sulphate.

In the preferred embodiment of the invention, the concentration of at least one silver salts in the antiseptic veterinary formulation is 1 to 1000 µmol/L.

The claimed invention relates to the antiseptic veterinary ointment containing nanosized silver particles and benzyl-dimethyl-[3-(myristoylamino)propyl]-ammonium chloride.

The claimed invention relates to the antiseptic veterinary gel containing nanosized silver particles and benzyl-dimethyl-[3-(myristoylamino)propyl]-ammonium chloride.

The claimed invention also relates to a method for treating rhinitis in animals, according to which the antiseptic veterinary formulation containing nanosized silver particles and benzyl-dimethyl-[3-(myristoylamino)propyl]-ammonium chloride is administered via the intranasal route from 2-5 times daily until complete resolution of clinical signs.

The claimed invention also relates to a method for treating conjunctivitis in animals, according to which the eye drops containing nanosized silver particles and benzyl-dimethyl-[3-(myristoylamino)propyl]-ammonium chloride are used 2-4 times a day until complete resolution of clinical signs.

The claimed invention also relates to a method for treating gingivitis in animals, according to which the gums are periodically treated with the antiseptic veterinary formulation containing nanosized silver particles and benzyl-dimethyl-[3-(myristoylamino)propyl]-ammonium chloride until complete resolution of clinical signs.

The claimed invention also relates to a method for treating enteritis in animals, according to which animals are fed with drinking water with the antiseptic veterinary formulation containing nanosized silver particles and benzyl-dimethyl-[3-(myristoylamino)propyl]-ammonium chloride until complete resolution of clinical signs.

The claimed invention also relates to a method of treating wounds in animals, according to which the wound is periodically treated with the antiseptic veterinary formulation containing nanosized silver particles and benzyl-dimethyl-[3-(myristoylamino)propyl]-ammonium chloride in the form of aqueous solution, gel or ointment chloride until complete resolution of clinical signs.

The claimed invention also relates to a method of treating mastitis, according to which the antiseptic veterinary formulation containing nanosized silver particles and benzyl-dimethyl-[3-(myristoylamino)propyl]-ammonium chloride is administered intracisternally 1 time a day until complete resolution of clinical signs.

The claimed invention also relates to a method of treating endometritis, according to which the antiseptic veterinary formulation containing nanosized silver particles and benzyl-dimethyl-[3-(myristoylamino)propyl]-ammonium chloride is administered to the uterus 1-2 times a day until complete resolution of clinical signs.

The claimed invention also relates to a method for preventing diarrhea and enteritis in animals, according to which the animals are fed with feed and drinking water with the antiseptic veterinary preparation containing nanosized silver particles and benzyldimethyl- [3-(myristoylamino)propyl]-ammonium chloride.

The claimed invention also relates to a method for preventing diarrhea and enteritis in suckling piglets, according to which the antiseptic veterinary preparation containing nanosized silver particles and benzyl-dimethyl-[3-(myristoylamino)propyl]-ammonium chloride is sprayed over mammary glands of breeding pigs.

The claimed invention also relates to a method of preventing postoperative complications, according to which post-surgical sutures are treated with antiseptic veterinary preparation containing nanosized silver particles and benzyl-dimethyl-[3-(myristoylamino)propyl]-ammonium chloride.

The invention is further described with the aid of the following examples of alternative embodiments thereof.

### Example 1

The antiseptic veterinary preparation was used in the form of a solution containing 0.01 wt% of benzyl-dimethyl-[3-(myristoylamino)propyl]-ammonium chloride and 10 µg/ml of nano-sized particles of silver. The solution was prepared according to the procedure described in Russian Federation patent No. 2419439 of May 27, 2011 Antibacterial Preparation and Method for the Production Thereof. In addition to the benzyl-dimethyl-[3-(myristoylamino)propyl]-ammonium chloride and nanosized silver particles, the antiseptic veterinary preparation contained water up to 100 wt%. The preparation was used for the treatment of rhinitis, conjunctivitis, gingivitis, enteritis, wounds, mastitis and endometritis in animals, and for the prevention of postoperative complications and gastrointestinal disorders.

It was found that the use of the preparation showed statistically-valid results such as a shortened time of treatment and reduction in the incidence of complications.

### Treatment of rhinitis

During the year, 133 (100%) cats with rhinitis were evaluated prior to commencing therapy. Viral rhinitis was observed in 43 (32.3%) cats, bacterial rhinitis - in 28 (21%) cats, mixed rhinitis - in 62 cats.

The antiseptic veterinary preparation was prescribed as a solution diluted 1:1 with distilled water in the form of intranasal drops: 2 drops into each nostril 3 times a day for 10-14 days. The first signs of clinical improvement were observed in the group with bacterial rhinitis in 22 (80%) cats on day 5-6. Complete resolution of clinical signs was observed in 2 (8%) cats on day 14-15, and 4 (12%) cats required the extension of the treatment until day twelve due to persistent mucous secretions.

The viral rhinitis group of cats showed the following results: of 43 cats, 22 (51.1%) cats improved general well-being and reduced the symptoms of rhinitis on day 14-15, the remaining 21 cats (48.8%) required the extension of the treatment due to persistent clinical signs.

In treating mixed rhinitis, the clinical improvement occurred in the same manner as in the bacterial rhinitis group, but in contrast to the first group, the treatment was longer.

None of the observed cats had complications after the disease. No side effects were observed in the group of subjects.

The control group received 0.05% aqueous chlorhexidine bigluconate solution prepared as the conventional solution diluted 1:1 with distilled water and 0.01% aqueous solution of benzyl-dimethyl-[3-(myristoylamino)propyl]-ammonium chloride. In the treatment of bacterial rhinitis, clinical improvement occurred on day10-12, i.e. significantly later than in the group which received the claimed antiseptic veterinary preparation, and in the treatment of viral rhinitis and mixed rhinitis no complete resolution of clinical signs was achieved.

### Treatment of conjunctivitis

78 cats with conjunctivitis were evaluated prior to commencing therapy. Unilateral conjunctivitis was observed in 47 (60.2%) cats and bilateral conjunctivitis was observed in 31 (39.7%) cats.

By etiological characteristics, cats with conjunctivitis, as well as cats with rhinitis, were divided into bacterial, viral and mixed groups. In this case, the claimed antiseptic veterinary preparation was used as eye drops prepared as a solution diluted 1:1 with distilled water.

The results showed that the use of the claimed preparation successfully resulted in complete recovery of 29 cats with bacterial conjunctivitis (37.1%) on day 8-9 by using the preparation 2 times a day 2 drops in each eye.

Viral conjunctivitis: 14 (17.9%) cats showed clinical improvements on day 10-11, 9 (64.2%) cats showed complete resolution of clinical signs when using the claimed preparation as monotherapy, 5 (35.7%) cats required the combination therapy.

In the mixed conjunctivitis group, 35 cats received the therapy, of which 28 (80%) cats showed clinical improvement on day 10-11, 7 (20%) cats required the combination therapy.

The control group received 0.05% aqueous chlorhexidine bigluconate solution and 0.01% solution of benzyl-dimethyl-[3-(myristoylamino)propyl]-ammonium chloride in the form of eye drops. Only the bacterial conjunctivitis group showed successful treatment results, but not earlier than day 12-14. No permanent effect was achieved in the mixed and viral conjunctivitis groups.

### Treatment of gingivitis

83 (100%) cats were enrolled to the gingivitis group. The cats with gingivitis were divided into two groups: the catarrhal gingivitis group characterized with gum surface lesions involving the superficial connective tissue of mucosa in which cats usually didn't refuse food, and the ulcers gingivitis group, in which cats most often refused food from the early days of disease manifestation. The catarrhal gingivitis group included 61 (73.4%) cats, and the second group included 22 (27.6%) cats, respectively. In the first case, when gums were treated with antiseptic veterinary preparation, clinical improvement in the form of disappearance of mucous membrane hyperemia occurred on day 5-6 in 59 (96.7%) cats, 3 cats required the extension of treatment up to 10 days. In the ulcers gingivitis group, of 22 cats 16 resumed eating on their own on day 7-8 treatment that can be attributed to epithelialization of ulcerative surfaces, which began on day 7-8 of the drug use. The claimed preparation was applied to the gum mucous. All animals with gingivitis received light diet without dry or other coarse feed.

In the control group, the gums were treated with 0.05% aqueous chlorhexidine bigluconate solution or 0.01% aqueous benzyl-dimethyl-[3-(myristoylamino)propyl]-ammonium chloride solution. No lasting treatment effects were observed.

### Treatment of enteritis

76 cats with clinical manifestations of enteritis were enrolled in the study group, stool culture was conducted in all cases. Among bacterial pathogens, Salmonella spp., Campylobacter spp., Yersinia enterocolitica were identified in 62 cases (82%). E. coli, Pentotricchomonas hominis, G. Lambia, Toxoplasma gondii, Chlamidya trachomatis, coronavirus were identified in the remaining cases (18%).

All cats received the claimed veterinary preparation in the form of a solution containing 5 ml preparation per 1 liter of drinking water. 30 cats refused to self-administer the preparation; therefore they were forced to drink the liquid solution 2 ml every 2 hours within 5 days. 56 (74%) cats showed clinical improvement on day 3-4 (decreased frequency of stool, improved appetite), complete stool normalization occurred in 49 (64.4%) cats advancing on day 3-4, in 7 (9.2%) on day 7-8. Of 76 cats, 20 (26.3%) required longer treatment up to 15-16 days.

A control group of animals received a 0.01% solution of benzyl-dimethyl-[3-(myristoylamino)propyl]-ammonium monohydrate chloride. The treatment effect was achieved, on average, on day 3.

### Treatment of wounds

Cats and dogs with bite, post-surgical and avulsed wounds were observed. In using the claimed veterinary preparation, epithelization occurred earlier than in the control group, on average, on day 2-3, no infectious complications were observed in 89% cases. The control group received a 0.01% benzyl-dimethyl-[3-(myristoylamino)propyl]-ammonium monohydrate chloride.

### Treatment of mastitis

20 black-and-white cows with serous, catarrhal, fibrinous and purulent mastitis were observed. Treatment of serous mastitis was conducted as follows: 10 ml of claimed veterinary preparation was administered intracisternally using a syringe into each affected quarter of the udder once daily, 10% CaCl2 solution combined with 40% glucose prepared in the form of 1:1 solution was administered intravenously in the volume of 300 ml on day land 3 of the therapeutic course, S.G. Isaev presacral block (0.5% Novocain solution at a dose of 1 mL/kg in adrectal tissue at the middle point between the root of the tail and anus once on day 1) was applied, Ichthyol ointment or camphor ointment was applied to the udder 2 times a day. The average duration of treatment was 3 days. In treating seropurulent mastitis, in addition 2000000 u/kg streptomycin and penicillin were administered intramuscularly every other day.

In the claimed veterinary preparation group the complete clinical recovery occurred 1-2 days earlier than in the control group. The control group of 10 cows received a multifaceted drug Multiject instead of the claimed veterinary preparation which contained procaine penicillin, sulphate streptomycin, sulfate neomycin and prednisone.

### Treatment of endometritis

For the treatment of endometritis in cows, 100 ml of claimed veterinary preparation was administered to the uterus 2 times a day until the complete resolution of all clinical signs, which occurred, on average, over 5 days. When using the claimed veterinary preparation, the complete clinical recovery occurred 2 days earlier, as an average, than in the control group. The control group received 0.01% solution of benzyl-dimethyl-[3-(miristoylamino)-propyl]-ammonium monohydrate chloride or 1:5000 furacilin solution in combination with intramuscular potassium salt of benzylpenicillin and streptomycin sulfate instead of the claimed veterinary preparation. In the control group, the endometritis developed into the chronic form in a number of cases.

### Prevention of enteritis

The claimed veterinary preparation heated to 35-40°C was sprayed over mammary glands of breeding pigs for the prevention of diarrhea and gastroenteritis in suckling piglets during feeding 2 times a day. After weaning, the piglets received the claimed veterinary preparation with food, at the rate of 2 ml per animal.

The use of the claimed veterinary preparation allowed to exclude the occurrence of the gastrointestinal disorders, while diarrhea developed in 15 percent of piglets in the control group. The control group did not receive any antidiarrheal preparations.

The results also showed that the claimed veterinary preparation can be used for prevention of gastrointestinal tract disorders in calves and chickens. The application of declared veterinary preparation reduced the likelihood of gastrointestinal tract disorders by 2.5 times in calves, 1.8 times in chickens.

### Examples Group 1

In Examples Group 1, the veterinary preparation was prepared similarly to Example 1, with the concentration of benzyl-dimethyl-[3-(miristoylamino) propyl]-ammonium chloride ranged from 0.002 to 0.05 wt%, the concentration of nanosized silver particles ranged from 0.1 to 100 µg/ml, silver salt was added to the solution in some examples, with silver nitrate or acetate, or citrate , or chloride, bromide, iodide, or sulphate used as the silver salts, with the concentration of silver salts ranging from 1 to 1000 µmol/l. Slightly soluble silver salts, such as silver chloride, bromide, and iodide silver sulfate were added to the solution in the form of powder or powder dispersed in water to form the corresponding colloidal solution. In addition to benzyl-dimethyl-[3-(miristoylamino)propyl]-ammonium chloride, nanosized silver particles and, in the number of cases, silver salts, the antiseptic veterinary preparation contained water up to 100 wt%.

The effectiveness of the obtained veterinary preparations was assessed in the same way as in example 1 for prophylaxis of postoperative complications, treatment and prevention of mastitis and endometritis, conjunctivitis, gingivitis, diarrhea, rhinitis, otitis, in pets and farm animals, including cattle, birds, cats, dogs, as well as in the treatment of open wounds and damaged skin. In all cases, the technical result has been achieved, which consisted in the statistically significant reduction in the average duration of treatment and in the incidence of complications.

## Claims

1. Antiseptic veterinary formulation containing nanosized silver particles and benzyl-dimethyl-[3-myristoylamino)propyl]-ammonium chloride.

2. Antiseptic veterinary preparation of Claim 1, **characterized in that** the concentration of benzyl-dimethyl-[3-(myristoylamino)propyl]-ammonium chloride in the antiseptic veterinary preparation ranges from 0.002 to 0.05 wt%.

3. Antiseptic veterinary preparation of Claim 1, **characterized in that** the concentration of nanosized silver particles in the antiseptic veterinary preparation ranges from 0.1 to 100 µg/ml.

4. Antiseptic veterinary preparation of Claim 1, **characterized in that** the antiseptic veterinary preparation additionally contains at least one salt of silver.

5. Antiseptic veterinary preparation of Claim 4, wherein the silver salt is selected from the group consisting of silver nitrate, acetate, citrate, chloride, bromide, iodide, and sulfate.

6. Antiseptic veterinary preparation of Claim 4, wherein the concentration of at least one salt of silver in antiseptic veterinary formulation is 1 to 1000 mmol/L.

7. Antiseptic veterinary formulation containing from 0.002 to 0.05 wt% benzyl-dimethyl-[3 (miristoylamino)propyl]-ammonium chloride, from 0.1 to 100 ug/ml of nanosized silver particles and water up to 100 wt.%.

8. Antiseptic veterinary formulation containing from 0.002 to 0.05 wt% benzyl-dimethyl-[3-(miristoylamino)propyl]-ammonium chloride, from 0.1 to 100 ug/ml of nanosized silver particles, from 1 to 1000 mmol/L a silver salt and water up to 100 wt%.

9. Antiseptic veterinary preparation according to Claim 8, **characterized in that** the silver salt is selected from the group consisting of silver nitrate, acetate, citrate, chloride, bromide, iodide, and sulfate.

10. Antiseptic veterinary ointment comprising nanosized silver particles and benzyl-dimethyl- [3-(miristoylamino)propyl] -ammonium chloride.

11. Antiseptic veterinary gel comprising nanosized silver particles and benzyl-dimethyl-[3-(miristoylamino)propyl] -ammonium chloride.

12. A method for treating rhinitis in animals, wherein the antiseptic veterinary formulation containing nanosized silver particles and benzyl-dimethyl-[3-(myristoylamino)propyl]-ammonium chloride is administered via the intranasal route 2-5 times daily until complete resolution of clinical signs.

13. A method for treating rhinitis in animal, wherein the antiseptic veterinary formulation is administered via the intranasal route 2-5 times daily according to any one of claims 1 to 9 until complete resolution of clinical signs.

14. A method for treating conjunctivitis in animals, wherein the eye drops containing nanosized silver particles and benzyl-dimethyl-[3-(myristoylamino)propyl]-ammonium chloride are used 2-4 times a day until complete resolution of clinical signs.

15. A method for treating conjunctivitis in animals, wherein the eye drops of any of Claims 1 to 9 are used 2-4 times a day until complete resolution of clinical signs.

16. A method of treating gingivitis in animals, wherein the gums are periodically treated with the antiseptic veterinary formulation comprising nanosized silver particles and benzyl-dimethyl-[3-(myristoylamino)propyl]-ammonium chloride until complete resolution of clinical signs.

17. A method for treating gingivitis in animals, wherein the gums are periodically treated with antiseptic veterinary preparation according to any of Claims 1 to 9 until complete resolution of clinical signs.

18. A method for treating enteritis in animals, wherein animals receive drinking water with the solution of antiseptic veterinary preparation comprising nanosized silver particles and benzyl-dimethyl-[3-(miristoylamino)propyl]-ammonium chloride until complete resolution of clinical signs.

19. A method for treating enteritis in animals, wherein animals receive drinking water with the solution of the antiseptic veterinary preparation of any of Claims 1 to 9 until complete resolution of clinical signs.

20. A method for treating wounds in animals, wherein the wound is treated periodically with antiseptic veterinary preparation comprising nanosized silver particles and benzyl-dimethyl-[3-(miristoylamino)propyl]-ammonium chloride in the form of an aqueous solution, gel or ointment until complete resolution of clinical signs.

21. A method of treating wounds in animals, wherein the wound is treated periodically with antiseptic veterinary preparation of any of Claims 1 to 9 in the form of aqueous solution, gel or ointment until complete resolution of clinical signs.

22. A method for treating mastitis, wherein the antiseptic veterinary preparation comprising nanosized silver particles and benzyl-dimethyl-[3-(miristoylamino)propylammonium chloride is administered intracisternally one time per day until complete resolution of clinical signs.

23. A method for treating mastitis, wherein the antiseptic veterinary preparation of any of Claims 1 to 9 one time per day is administered intracisternally until complete resolution of clinical signs.

24. A method for treatment of endometritis, wherein the antiseptic veterinary preparation comprising nanosized silver particles and benzyl-dimethyl-[3-(miristoylamino)propyl]-ammonium chloride is administered to the uterus 1 to 2 times daily until complete resolution of clinical signs.

25. A method for treatment of endometritis, wherein the antiseptic veterinary preparation of any of Claims 1 to 9 is administered to the uterus 1 to 2 times daily until complete resolution of clinical signs.

26. A method for preventing diarrhea and enteritis in animals, wherein the animals receive food or drinking water with the antiseptic veterinary preparation comprising nanosized silver particles and benzyl-dimethyl-[3-(miristoylamino)propyl]-ammonium chloride.

27. A method for preventing diarrhea and enteritis in animals wherein the animal with the feed or drinking water injected antiseptic veterinary preparation according to any one of claims 1 to 9.

28. A method for preventing diarrhea and enteritis in piglets, wherein the antiseptic veterinary preparation comprising nanosized silver particles and benzyl-dimethyl-[3-(miristoylamino)propyl]-ammonium chloride is sprayed over mammary glands of breeding pigs.

29. A method for preventing diarrhea and enteritis in piglets, wherein the antiseptic veterinary preparation of any of Claims 1 to 9 is sprayed over mammary glands of breeding pigs.

30. A method for preventing postoperative complications, wherein the post-surgical sutures are treated with the antiseptic veterinary preparation comprising nanosized silver particles and benzyl-dimethyl-[3-(miristoylamino)propyl]-ammonium chloride.

31. A method for preventing postoperative complications, wherein the post-surgical sutures are treated with the antiseptic veterinary preparation of any of Claims 1 to 9.
